# EUROPEAN PATENT SPECIFICATION

(11) **EP 0 446 699 B1**
(45) Date of publication and mention of the grant of the patent: **31.05.2000**
(21) Application number: 91102796.9
(22) Date of filing: 26.02.1991
(51) Int. Cl.: C07K 5/06, C07C 271/10, C07C 271/20, C07C 271/24, C07C 275/20, C07C 275/26, C07C 279/04, C07C 279/16, C07C 281/02, C07C 281/06, C07C 281/16, C07C 335/06, C07C 337/06, A61K 31/155, A61K 31/17, A61K 31/27

(54) **Inhibitors of nitric oxide biosynthesis**
Inhibitoren für die Stickoxid-Biosynthese
Inhibiteurs de la biosynthèse de l'oxyde nitrique

(30) Priority: 26.02.1990 US 485109; 19.09.1990 US 585349
(43) Date of publication of application: 18.09.1991
(73) Proprietor: MERRELL PHARMACEUTICALS INC., Cincinnati, Ohio 45215-6300 (US)
(72) Inventor: Whitten, Jeffrey P., Cincinnati, Ohio 45241 (US); Lambert, Laurie E., West Chester, Ohio 45069 (US); McDonald, Ian A., Loveland, Ohio 45140 (US); Doherty, Niall S., Lyme, Connecticut 06371 (US)
(74) Representative: VOSSIUS & PARTNER

(56) References cited:
- EP-A- 0 196 841
- EP-A- 0 230 037
- EP-A- 0 277 561
- WO-A-91/04023
- CHEMICAL ABSTRACTS vol. 84, January 1976, page 20, abstract no. 197d, Columbus, Ohio, US; G.P. WHEELER et al.: "Carbamoylation of amino acids, peptides, and proteins by nitrosoureas" & Cancer Res. 1975, vol. 35, no. 11, part 1, pages 2974-2984
- CHEMICAL ABSTRACTS vol. 83, no. 25, 22 December 1975, page 421, abstract no. 206526y, Columbus, Ohio, US; A. TURAN et al.: "Removal of the nitro group from nitroarginine and nitroarginine peptides" & Acta Chim. Acad. Sci. Hung. 1975, vol. 85, no. 3, pages 327-332 & formula index 1975, vol. 83, page 539F, column 3, lines 79-81
- TETRAHEDRON LETTERS vol. 29, no. 47, 1988, pages 6183,6184, GB; V. TOLMAN et al.: "Unsaturatd amino acids: Synthesis of trans-3,4-didehydro analogues of L-crnithine and L-arginine"
- Br J Pharmacol 99 (1990) 408-412

## Description

The present invention is directed to a new class of arginine derivatives which are useful in inhibiting the biosynthesis of nitric oxide from L-arginine. A further aspect of the invention is directed to compositions containing these arginine derivatives which can be useful in the treatment of a number of disease states such as, for example, conditions associated with low blood pressure and to the treatment of inflammatory diseases and stroke.

It has recently been discovered that nitric oxide mediates a number of physiological processes. It serves as a signal transduction mechanism in the central nervous system, is intimately involved with the regulation of blood pressure, and is produced by activated inflammatory cells thereby playing a role in pathological inflammatory conditions.

Garthwaite et al. and Stevens reported that stimulation of the glutamate site of the NMDA receptor complex leads to the release of nitric oxide. The nitric oxide stimulates the guanylate cyclase present in the CNS which in turns leads to an increase in cyclic GMP levels. A great deal of attention has been focused upon these excitatory amino acid receptors because it is believed that they are associated with learning and development. It is also believed that over-stimulation of these receptors is associated with a number of disease states such as, for example, epilepsy, stroke, and neurodegenerative diseases such as senile dementia, etc. *Nature*, 336, pages 308-309 and 385-388 (November 1988).

Knowles et al. reported the mechanism by which the nitric oxide is formed. An enzymatic reaction occurs in which L-arginine is converted into nitric oxide and citrulline. Knowles et al. also reported that the presence of N-monomethyl-L-arginine inhibited this enzymatic transformation. *Proc. Natl. Acad. Sci. USA*, 86, pages 5159-5161 (July 1989).

Moncada et al. provided a review of the information which had been published to date regarding the physiological role of nitric oxide. Moncada et al. described the work of Knowles and Garthwaite regarding the role of nitric oxide in the CNS and postulated that nitric oxide is involved in seizures and other disease states associated with excessive excitatory neurotransmission. *Biochemical Pharmacology*, 38, No. 11, pages 1709-1715, (1989).

Moncada et al. reported that nitric oxide is associated with the regulation of blood pressure. These authors reported that an enzymatic pathway exists in the vasculature by which L-arginine is converted into nitric oxide. *Nature*, 333, pages 664-666 (1988). The nitric oxide relaxes the smooth muscles of the vasculature, producing dilation of these vessels which results in a lowering of blood pressure. Inhibition of nitric oxide synthesis by the administration of N-monomethyl-L-arginine produces a marked rise in blood pressure in test animals. *Proc. Natl. Acad. Sci. USA*, 86, pages 3375-3378 (1989).

Moncada et al. also reviewed earlier literature which reported that nitric oxide can be produced in large amounts by macrophages and polymorphonuclear leucocytes (PMN) and is associated with the effector functions of the cells. N-monomethyl-L-arginine inhibits the synthesis of nitric oxide by these cells.

Br. J. Pharmacol. (1990), 99, 408-412 discloses L-N^{G}-nitro arginine and suggests that it inhibits endothelial nitric oxide biosynthesis like L-N^{G}-monomethyl arginine.

Thus, it is the object of the present application to provide compounds capable of inhibiting the biosynthesis of nitric oxide. They would provide a new treatment for a number of disease states and could be utilized in the treatment of conditions associated with low blood pressure such as, for example, shock. They could also be used to treat conditions caused by over-activity of the NMDA receptor complex such as epilepsy, stroke, neurodegenerative diseases, etc. They could also be used to treat conditions associated with excessive production of nitric oxide by macrophages and polymorphonuclear leucocytes such as arthritis, cirrhosis, transplant rejections, etc.

This object has been achieved by the surprising finding that the biosynthesis of nitric oxide can be inhibited by the following class of arginine derivatives represented by the formula I in which:
W is represented by -CH₂-NH-; and
R is represented by OM in which M is H,
Z is represented by NR₁, wherein R₁ represents H,
and X is represented by cyclopropyl.

The compounds encompassed by Formula I inhibit the biosynthesis of nitric oxide. They prevent the bio-conversion of L-arginine into nitric oxide and citrulline. Thus these compounds will be useful in the treatment of a number of disease states in which excessive levels of nitric oxide are implicated.

As used in this application: the term, "cyclopropyl" refers to the following substituent:

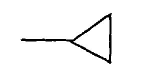

Since the compounds of Formula I are derivatives of the amino acid arginine, they can exist as acid addition salts, basic addition salts, as zwitterions, or as their free base. Thus the term "pharmaceutically acceptable salts" should be construed as encompassing pharmaceutically acceptable acid additions salts, pharmaceutically acceptable basic addition salts or the zwitterion form of the molecule.

The expression "pharmaceutically acceptable acid addition salts" is intended to apply to any non-toxic organic or inorganic acid addition salt of the base compounds represented by Formula I or any of its intermediates. Illustrative inorganic acids which form suitable salts include hydrochloric, hydrobromic, sulphuric, and phosphoric acid and acid metal salts such as sodium monohydrogen orthophosphate, and potassium hydrogen sulfate. Illustrative organic acids which form suitable salts include the mono-, di-, and tricarboxylic acids. Illustrative of such acids are for example, acetic, glycolic, lactic, pyruvic, malonic, succinic, glutaric, fumaric, malic, tartaric, citric, ascorbic, maleic, hydroxymaleic, benzoic, hydroxy-benzoic, phenylacetic, cinnamic, salicylic, 2-phenoxy-benzoic, p-toluenesulfonic acid, and sulfonic acids such as methane sulfonic acid and 2-hydroxyethane sulfonic acid. Such salts can exist in either a hydrated or substantially anhydrous form.

The expression "pharmaceutically acceptable basic addition salts" is intended to apply to any non-toxic organic or inorganic basic addition salt of the compounds represented by Formula I or any of its intermediates. Illustrative bases which form suitable salts include alkali metal or alkaline-earth metal hydroxides such as sodium, potassium, calcium, magnesium, or barium hydroxides; ammonia, and aliphatic, alicyclic, or aromatic organic amines such as methylamine, dimethylamine, trimethylamine, and picoline.

All of the compounds of Formula I exist as optical isomers. Those compounds of Formula I in which X is represented by a butadiene will exist as stereoisomers as well. Those compounds of Formula I which are in the L-configuration are preferred due to their superior potency. However, any reference to the compounds of Formula I or to any of their intermediates should be construed as referring to either an optical isomer, an enantiomer, or to a racemic mixture. The specific optical isomers and enantiomers can be separated and recovered by techniques known in the art such as chromatography on chiral stationary phases or resolution via chiral salt formation and subsequent separation by selective crystallization. Alternatively utilization of a specific optical isomer or enantiomer as the starting material will produce the corresponding isomer or enantiomer as the final product.

The compounds of Formula I exist as tautomers. The geminal nitrogen atoms will exist in a state of equilibrium in which they share a pair of electrons. Any reference to the compounds of Formula I should be construed as encompassing any of these tautomers.

The compounds of Formula I can be made using techniques known in the art. Reaction Scheme I shows one method by which these compounds can be produced.

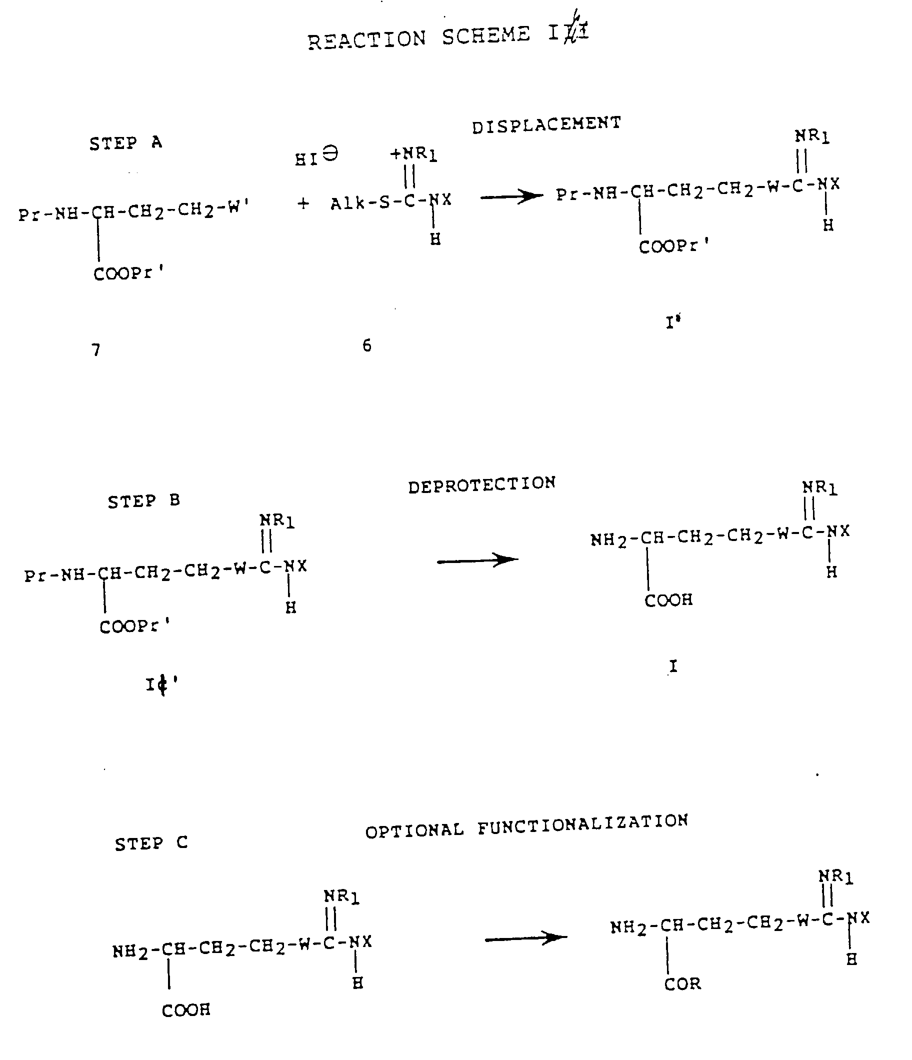

In Step A, a displacement reaction is carried out between a functionalized amino acid as described by structure (7) and an amidino derivative as described by structure (6). In structure (7), W' is represented by -CH₂-NH₂, and Pr and Pr' may both be hydrogen or copper. Alternatively Pr' is t-butyl while Pr is t-butyloxycarbonyl. In structure (6), Alk is represented by a C₁₋₄ alkyl, and R₁ and X are as defined in Formula I. This displacement reaction produces the optionally protected compound which has been depicted as I'. In step B, the protecting groups, if present, are removed via an acidic hydrolysis.

The displacement reaction of Step A can be carried out using techniques well known in the art. The appropriate functionalized amino acid of structure (7) is one in which W' corresponds to the substituent which is desired at this position in the final product. The meanings for Pr and Pr' should be complementary (ie. both should be hydrogen, organic residues or copper). The appropriate amidine is one in which X is represented by the substituent which is desired in the final product. The linear C₁₋₄ alkyl represented by Alk is not retained in the final product, and thus its identity is immaterial.

The particular manner in which the displacement reaction is carried out depends upon the particular substituent which is present at the Pr and Pr' position. If Pr and Pr' are represented by organic protecting groups, then the reaction is carried out by contacting approximately equivalent amounts of the functionalized amino acid and the amidino-derivative in an aprotic solvent such as dimethylformamide at a temperature range of from room temperature to about 80°C. The reaction is typically allowed to proceed for a period of time ranging from about 8 hours to about 120 hours. Upon completion of the reaction, the functionalized amino acid of formula I' can be recovered by either concentration or extraction as is known in the art. It can then be optionally be purified by flash chromatography. In Step B these protecting groups are removed by an acidic hydrolysis. This hydrolysis can be carried out in the same manner as the deprotection reaction of Reaction Scheme I. The deprotected compound of Formula I can be purified by either ion exchange chromatography or by preparative C₁₈ reverse chromatography. Likewise, the appropriate R substituent may be placed on the compound of Formula I utilizing the same methods as taught in Reaction Scheme I.

If Pr and Pr' are represented by either hydrogen atoms or by a copper complex then the displacement reaction is carried out in the following manner. Approximately equivalent amounts of the functionalized amino acid of structure (7) and the amidino derivative of structure (6) are contacted in a dilute solution of an inorganic base such as sodium hydroxide. The displacement reaction is carried out at about 40°C for a period of time ranging from about 8 to 120 hours. The desired product of Formula I can then be recovered from the reaction by either extraction or concentration as is known in the art. This crude product can then be purified by ion exchange chromatography or by preparative C₁₈ reverse chromatography.

Methods for producing the functionalized amino acids of structure (7) are known in the art. For example, the preparation of canaline is described in *J. Am. Chem. Soc*., 79, 1222 (1957).

The following examples represent typical syntheses as described above in Scheme I. These examples are intended to be illustrative only and are not intended to limit the scope of the present invention in any way.

### Example 1

### Preparation of N⁵-[Imino(cyclopropyl)methyl]-L-ornithine, flavinate

This example also demonstrates the methodology of Reaction Scheme I.
t-Butyl isothiocyanate (11.6ml, 0.1mol) and cyclopropylamine (6.9ml, 0.1mol) were mixed in toluene (200ml) and stirred at room temperature overnight. The resulting mixture was evaporated to a residue and crystallized from cyclohexane to yield N-cyclopropyl-N-butyl thiourea. N-cyclopropyl-N-t-butyl thiourea (5g) was suspended in 5M HCl (50ml) and heated to 80°C for 10 minutes. The resulting solution was cooled, poured into a saturated sodium bicarbonate (250ml), and extracted with ethyl acetate (2x125ml). The solution was dried (MgSO₄), evaporated to a residue and crystallized from ethyl acetate to yield a white solid, cyclopropyl thiourea, 110mg.
Cyclopropyl thiourea (0.65g, 0.0056mol) and iodomethane (1.2g, 0.0085mol) were mixed in acetone, heated to boiling and refluxed for 15 minutes. The reaction was cooled and blown to an oil. Ethyl acetate (10ml) was added, and the resulting mixture was blown to a white solid. L-Ornithine·HCl (0.94g, 0.0056mol) and 1M NaOH (15ml) were added and the resulting reaction warmed to 40°C and stirred under a blanket of N₂ for 16 hours. The reaction mixture was cooled to room temperature, acidified with acetic acid and blown with a stream of N₂ to 1/2 volume. Flavianic acid (1g) in water (10ml) was added and the resulting solid filtered, dried and recrystallized from boiling water to yield 1.4g of the title compound which appeared as a yellow solid. ¹HNMR (d₆DMSO) 0.52 (2,m) 0.82 (2,m) 1.45 (2,m) 1.78 (2,m) 2.5 (1,m) 3.15 (2,m) 3.85 (1,m) 7.8 (1,m) 8.1-8.8 (2,m) 9.2 (1,s). Anal Calcd. for C₉H₁₈N₄O₂·C₁₀H₆N₂O₈S.1/2H₂O: C, 41.97; H, 4.64; N, 15.76. Found C, 42.67; H, 4.56; N, 15.76.

An alternative method for producing the compounds of Formula I is to carry out a displacement reaction between a functionalized amino acid as described by structure (7) in which Pr and Pr' are represented by protecting groups and an amino nitrile of the Formula, X-NH-CN, in which X is as defined in Formula I. This displacement reaction produces the protected compound of Formula I' disclosed above in Reaction Scheme I. This displacement can be carried out by contacting equivalent amounts of the reactants in an aprotic solvent such as toluene or benzene at a temperature of about -5°C to reflux for a period of time ranging from about 1 hour to 16 hours. The compound of Formula I' can be recovered by either extraction or concentration. It can then be purified by ion exchange chromatography or preparative C₁₈ reverse phase chromatography. The desired compounds of Formula I can then be produced via the deprotection reaction.

As noted above it has been demonstrated that nitric oxide relaxes the smooth muscles of the vasculature, producing a dilatory effect upon these vessels which results in a reduction in blood pressure. Since the compounds of Formula I inhibit the biosynthesis of nitric oxide, the administration of the compounds of Formula I will produce a rise in blood pressure and thus can be used to treat conditions associated with low blood pressure. Representative examples of such conditions include shock, endotoxic shock, hypovolemic shock, cardiogenic shock.

In order to exhibit this hypertensive effect it is necessary that the compounds be administered in an amount sufficient to inhibit the biosynthesis of nitric oxide. The dosage range at which these compounds exhibit this hypertensive effect can vary widely depending upon the particular disease being treated, the severity of the patient's disease, the patient, the particular compound being administered, the route of administration, and the presence of other underlying disease states within the patient, etc. Typically the compounds exhibit their hypertensive effects at a dosage range of from about 0.1 mg/kg/day to 500mg/kg/day. Repetitive daily administration may be desirable and will vary according to the conditions outlined above. Typically, the compounds will be administered from 1-4 times daily.

The ability of these compounds to inhibit the biosynthesis of nitric oxide can be demonstrated by in vitro assays known in the art. Igengar et al. *Proc. Natl. Acad. Sci*., 84, pages 6369-6373 (Sept. 1987). In this assay macrophages are exposed to bacterial lipopolysaccharides and Interferon-gamma, which stimulates the macrophages to produce nitric oxide. The compounds of Formula I will either inhibit or decrease production of nitric oxide by the macrophages.

It is well known in the art that excessive activity by macrophages is implicated in a number of inflammatory diseases such as, for example, rheumatoid arthritis, inflammatory bowel disease, multiple sclerosis, cirrhosis of the liver and lungs, sarcoidosis, and granulomatous lesions. In a recent review, Collier et al. stated that it is currently believed that the cytotoxic activity of macrophages is due their ability to produce nitric oxide. *Trends in Pharmacological Sciences*, 10(11), page 123, (1989) Thus the compounds of the instant invention will either inhibit or decrease the amount of nitric oxide which is produced by the macrophages and thus will be useful in the treatment of these disease states associated with excessive activity of macrophages. The compounds will also be useful in preventing transplant rejection by macrophages.

In order to exhibit this beneficial effect in inflammatory diseases, it is necessary that the compounds of Formula I be administered in a quantity sufficient to inhibit the production of nitric oxide by macrophages. The dosage range at which these compounds exhibit this effect can vary widely depending upon the particular disease being treated, the severity of the patient's disease, the patient, the particular compound being administered, the route of administration, and the presence of other underlying disease states within the patient, etc. Typically the compounds exhibit their therapeutic effect at a dosage range of from about 0.1 mg/kg/day to about 500 mg/kg/day for any of the diseases or conditions listed above. Repetitive daily administration may be desirable and will vary according to the conditions outlined above.

The ability of these compounds to inhibit the generation of cyclic GMP within the central nervous system can be demonstrated by procedures known in the art such as Baron et al. *Journal of Pharmacol. and Exp. Therap*., 250, pages 162-169 (1989). In this test, mice are administered harmaline which produces a rise in cyclic GMP levels with the cerebella of the CNS. The pre-administration of one of the compounds of Formula I will block the rise in cyclic GMP levels that is typically associated with harmaline administration.

It has been demonstrated that stimulation of the NMDA receptor complex leads to the release of nitric oxide which in turn stimulates guanylate cyclase which in turn produces a rise in cyclic GMP levels. Since the compounds of Formula I will inhibit the production of cyclic GMP and thus will negate the physiological effects typically associated with stimulation of the NMDA receptor complex, these compounds will be useful in the treatment of disease states associated with overstimulation of these excitatory neurons. Overstimulation of the NMDA receptor complex has been associated with seizures and thus the compounds of Formula I exhibit anti-convulsant properties and are useful in the treatment of epilepsy. They are useful in the treatment of grand mal seizures, petit mal seizures, psychomotor seizures, and autonomic seizures.

Overstimulation of the NMDA receptor complex has also been associated with the neurotoxicity associated with ischemic, hypoxic, traumatic, or hypoglycemic conditions. Representative examples of such ischemic, hypoxic, or hypoglycemic conditions include strokes or cerebrovascular accidents, carbon monoxide poisoning, hyperinsulinemia, cardiac arrest, drownings, suffocation, and neonatal anoxic trauma. The compounds should be administered to the patient within 24 hours of the onset of the hypoxic, ischemic, or hypoglycemic condition in order for the compounds to effectively minimize the CNS damage which the patient will experience.

The compounds of Formula I are also useful in the treatment of neurodegenerative diseases such as Huntington's disease, Alzheimer's disease, senile dementia, glutaric acidaemia type I, multi-infarct dementia, and neuronal damage associated with uncontrolled seizures. The administration of these compounds to a patient experiencing such a condition will serve to either prevent the patient from experiencing further neurodegeneration or it will decrease the rate at which the neurodegeneration occurs.

As is apparent to those skilled in the art, the compounds will not correct any CNS damage that has already occurred as the result of either disease or a lack of oxygen or sugar. As used in this application, the term "treat" refers to the ability of the compounds to prevent further damage or delay the rate at which any further damage occurs.

The dosage range at which these compounds will exhibit their effect upon excitatory neurotransmission can vary widely depending upon the particular disease being treated, the severity of the patient's disease, the patient, the particular compound being administered, the route of administration, and the presence of other underlying disease states within the patient, etc. Typically the compounds exhibit their therapeutic effect at a dosage range of from about 0.1 mg/kg/day to about 500 mg/kg/day for any of the diseases or conditions listed above. Repetitive daily administration may be desirable and will vary according to the conditions outlined above.

As used in this application:
a) the term "patient" refers to warm blooded animals such as, for example, guinea pigs, mice, rats, cats, rabbits, dogs, monkeys, chimpanzees, and humans;
b) the term "treat" refers to the ability of the compounds to either relieve, alleviate, or slow the progression of the patient's disease;
c) the term "neurodegeneration" refers to a progressive death and disappearance of a population of nerve cells occurring in a manner characteristic of a particular disease state and leading to brain damage.
d) the term "inflammatory disease" refers to a condition in which activation of leukocytes leads to an impairment of normal physiologic function. Representative examples of such conditions include rheumatoid arthritis, inflammatory bowel disease, sepsis, and adult respiratory distress syndrome.
e) the term "hypotensive condition" refers to a condition in which blood pressure is lowered to a point to which normal physiologic functioning is impaired.

The compounds of the present invention may be administered by a variety of routes. They are effective if administered orally. The compounds may also be administered parenterally (i.e., subcutaneously, intravenously, intramuscularly, intraperitoneally, or intrathecally).

Pharmaceutical compositions can be manufactured utilizing techniques known in the art. Typically an inhibitory amount of the compound will be admixed with a pharmaceutically acceptable carrier.

For oral administration, the compounds can be formulated into solid or liquid preparations such as capsules, pills, tablets, lozenges, melts, powders, suspensions, or emulsions. Solid unit dosage forms can be capsules of the ordinary gelatin type containing, for example, surfactants, lubricants and inert fillers such as lactose, sucrose, and cornstarch or they can be sustained release preparations. In another embodiment, the compounds of Formula I can be tableted with conventional tablet bases such as lactose, sucrose, and cornstarch in combination with binders, such as acacia, cornstarch, or gelatin, disintegrating agents such as potato starch or alginic acid, and a lubricant such as stearic acid or magnesium stearate. Liquid preparations are prepared by dissolving the active ingredient in an aqueous or non-aqueous pharmaceutically acceptable solvent which may also contain suspending agents, sweetening agents, flavoring agents, and preservative agents as are known in the art.

For parenteral administration the compounds may be dissolved in a physiologically acceptable pharmaceutical carrier and administered as either a solution or a suspension. Illustrative of suitable pharmaceutical carriers are water, saline, dextrose solutions, fructose solutions, ethanol, or oils of animal, vegetative, or synthetic origin. The pharmaceutical carrier may also contain preservatives, buffers, etc., as are known in the art. When the compounds are being administered intrathecally, they may also be dissolved in cerebrospinal fluid as is known in the art.

The compounds may also be admixed with any inert carrier and utilized in laboratory assays in order to determine the concentration of the compounds within the serum, urine, etc., of the patient as is known in the art.

## Claims

1. Compound of the formula I 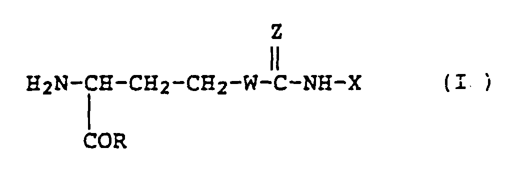 in which
X is represented by cyclopropyl,
R is represented by OM in which M is represented by H,
Z is represented by NR₁, in which R₁ is represented by H, and
W is represented by -CH₂-NH-.

2. A process for producing the compound of formula I comprising:
a) carrying out a displacement reaction between a functionalized amino acid as described by structure (7) 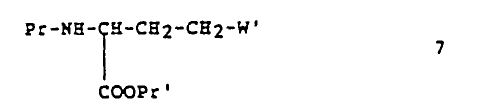 in which W' is -CH₂-NH₂ and in which Pr and Pr' are hydrogen or protecting groups and an amidino derivative as described by structure (6) 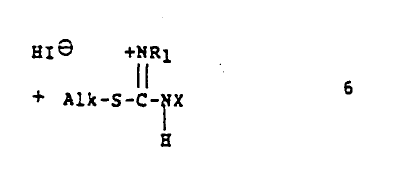 in which Alk is represented by C₁₋₄ alkyl and R₁, and X are as defined in claim 1, thereby producing the compound of formula I', 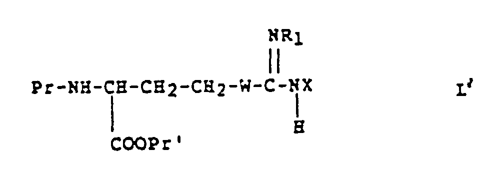
b) removing the protecting groups, if present, via an acidic hydrolysis.

3. A pharmaceutical composition comprising the compound of claim 1.

4. Use of a compound according to claim 1 for the preparation of a pharmaceutical composition.

5. Use according to claim 4 wherein the composition is useful for inhibiting the biosynthesis of nitric oxide.

6. Use according to claim 4 wherein the composition is useful for the treatment of hypotensive conditions.

7. Use according to claim 4 wherein the composition is useful for the treatment of inflammatory diseases.

8. Use according to claim 4 wherein the composition is useful for the treatment of epilepsy.

9. Use according to claim 4 wherein the composition is useful for the treatment of neurotoxicity associated with ischemic, hypoxic, traumatic or hypoglysemic conditions.

10. Use according to claim 4 wherein the composition is useful for the treatment of neurodegenerative diseases.

11. A method for the preparation of a pharmaceutical composition comprising combining a compound as defined in claim 1 with a pharmaceutically acceptable carrier.

## Patentansprüche

1. Verbindung der Formel I 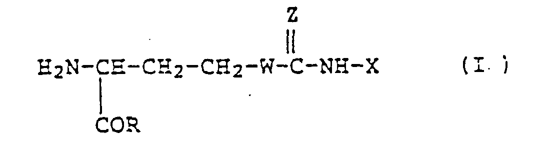 in der
X einen Cyclopropylrest darstellt,
R einen Rest OM wiedergibt, in dem M ein Wasserstoffatom darstellt,
Z einen Rest NR₁ wiedergibt, in dem R₁ ein Wasserstoffatom darstellt, und
W eine Gruppe -CH₂-NH- wiedergibt.

2. Verfahren zum Herstellen der Verbindung der Formel I, umfassend:
a) Ausführen einer Substitutionsreaktion zwischen einer funktionalisierten Aminosäure, wie durch Struktur (7) beschrieben, 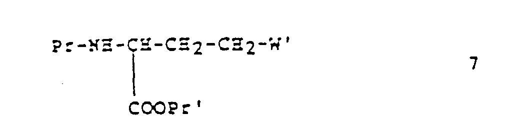 in der W' eine Gruppe -CH₂-NH₂ bedeutet und in der Pr und Pr' Wasserstoffatome oder Schutzgruppen darstellen, und einem Amidinoderivat, wie durch Struktur (6) beschrieben 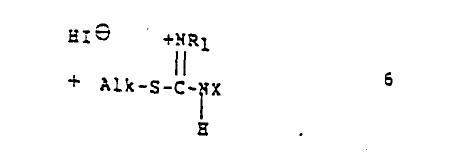 in der Alk einen C₁₋₄-Alkylrest wiedergibt und R₁ und X wie in Anspruch 1 definiert sind, wobei die Verbindung der Formel I' hergestellt wird, 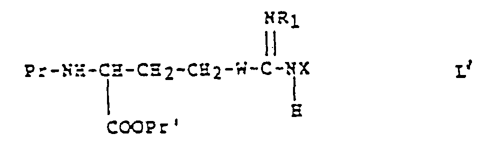
b) Entfernen der Schutzgruppen, falls anwesend, über eine saure Hydrolyse.

3. Arzneimittel, das die Verbindung des Anspruchs 1 umfaßt.

4. Verwendung einer Verbindung nach Anspruch 1 zur Herstellung eines Arzneimittels.

5. Verwendung nach Anspruch 4, wobei die Verbindung zur Hemmung der Stickoxid-Biosynthese verwendbar ist.

6. Verwendung nach Anspruch 4, wobei die Verbindung zur Behandlung hypotensiver Zustände verwendbar ist.

7. Verwendung nach Anspruch 4, wobei die Verbindung zur Behandlung entzündlicher Erkrankungen verwendbar ist.

8. Verwendung nach Anspruch 4, wobei die Verbindung zur Behandlung von Epilepsie verwendbar ist.

9. Verwendung nach Anspruch 4, wobei die Verbindung zur Behandlung von Neurotoxizität verwendbar ist, die mit ischämischen, hypoxischen, traumatischen oder hypoglykämischen Zuständen verbunden ist.

10. Verwendung nach Anspruch 4, wobei die Verbindung zur Behandlung neurodegenerativer Erkrankungen verwendbar ist.

11. Verfahren zur Herstellung eines Arzneimittels, das das Mischen einer Verbindung, wie in Anspruch 1 definiert, mit einem pharmazeutisch verträglichen Träger umfaßt.

## Revendications

1. Composé de formule (I) 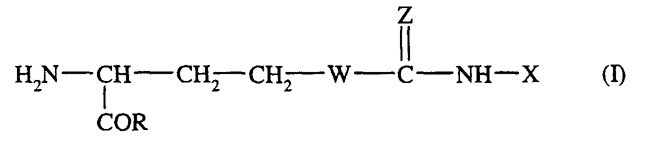 dans laquelle
X représente cyclopropyle,
R représente OM où M représente H,
Z représente NR₁, où R₁ représente H et
W représente -CH₂-NH-.

2. Procédé pour la production du composé de formule I comprenant :
a) la mise en oeuvre d'une réaction de déplacement entre un aminoacide fonctionnalisé tel que représenté par la structure (7) 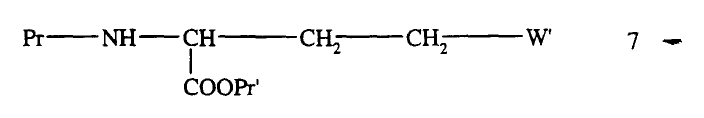 dans laquelle W' est -CH₂-NH₂ et dans laquelle Pr et Pr' sont des atomes d'hydrogène ou des groupes protecteurs et un dérivé amidino tel que représenté par la structure (6) 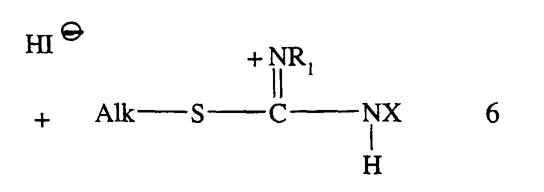 dans laquelle Alk représente un alkyle en C₁₋₄ et R₁ et X sont tels que définis dans la revendication 1, avec ainsi production du composé de formule I', 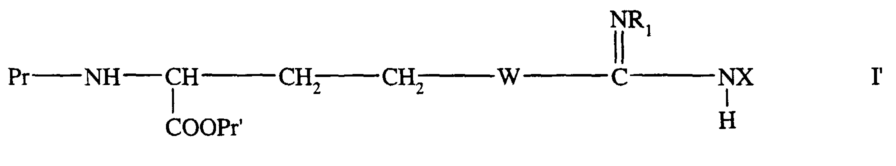
b) l'élimination des poupes protecteurs, s'il en existe, par hydrolyse acide.

3. Composition pharmaceutique comprenant le composé selon la revendication 1.

4. Utilisation d'un composé selon la revendication 1 pour la préparation d'une composition pharmaceutique.

5. Utilisation selon la revendication 4, dans laquelle la composition est utile pour l'inhibition de la biosynthèse de l'oxyde nitrique.

6. Utilisation selon la revendication 4, dans laquelle la composition est utile pour le traitement d'états pathologiques hypotensifs.

7. Utilisation selon la revendication 4, dans laquelle la composition est utile pour le traitement de maladies inflammatoires.

8. Utilisation selon la revendication 4, dans laquelle la composition est utile pour le traitement de l'épilepsie.

9. Utilisation selon la revendication 4, dans laquelle la composition est utile pour le traitement d'une neurotoxicité associée à des états ischémiques, hypoxiques, traumatiques ou hypoglycémiques.

10. Utilisation selon la revendication 4, dans laquelle la composition est utile pour le traitement de maladies neurodégénératives.

11. Méthode pour la préparation d'une composition pharmaceutique comprenant la combinaison d'un composé tel que défini dans la revendication 1 avec un support pharmaceutiquement acceptable.
